(19) Europäisches Patentamt / European Patent Office / Office européen des brevets

(11) **EP 3 383 353 B1**

(12) **EUROPEAN PATENT SPECIFICATION**

(45) Date of publication and mention
of the grant of the patent:
**30.10.2019 Bulletin 2019/44**

(21) Application number: **16794351.3**

(22) Date of filing: **11.11.2016**

(51) Int Cl.:
*A61K 8/25* *(2006.01)*          *A61Q 11/00* *(2006.01)*
*A61K 8/02* *(2006.01)*          *A61K 8/19* *(2006.01)*

(86) International application number:
**PCT/EP2016/077405**

(87) International publication number:
**WO 2017/093002 (08.06.2017 Gazette 2017/23)**

(54) **ORAL CARE COMPOSITION FOR REMINERALISATION AND WHITENING OF TEETH**

MUNDPFLEGEZUSAMMENSETZUNGEN ZUR REMINERALISIERUNG UND AUFHELLUNG DER ZÄHNE

COMPOSITION DE SOINS BUCCAUX POUR REMINÉRALISATION ET BLANCHIMENT DES DENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.12.2015 EP 15198112**
**25.01.2016 US 201662286491 P**

(43) Date of publication of application:
**10.10.2018 Bulletin 2018/41**

(73) Proprietor: **Omya International AG**
**4665 Oftringen (CH)**

(72) Inventors:
• **BUDDE, Tanja**
**4805 Brittnau (CH)**

• **GERARD, Daniel, E.**
**4058 Basel (CH)**
• **GANE, Patrick, A., C.**
**4852 Rothrist (CH)**

(74) Representative: **Chwalka, Bettina**
**Maiwald Patentanwalts- und**
**Rechtsanwaltsgesellschaft mbH**
**Elisenhof**
**Elisenstraße 3**
**80335 München (DE)**

(56) References cited:
**WO-A1-2015/140308          WO-A1-2015/150011**

Remarks:
The file contains technical information submitted after the application was filed and not included in this specification

EP 3 383 353 B1

**Description**

The present invention relates to a new oral care composition and its use for remineralisation and whitening of teeth.

Tooth enamel is the hardest substance in the human body and contains about 96 wt.-% minerals, wherein the remaining is composed of water and organic material. The primary mineral of enamel is hydroxylapatite, which is a crystalline calcium phosphate. Enamel is formed on the tooth while the tooth is developing within the gum, before it erupts into the mouth.

Its high mineral content makes the enamel, however, very vulnerable to a demineralisation process, which is especially triggered by the consumption of acidic drinks and sweets. Remineralisation of teeth can repair damage to the tooth to a certain degree but damage beyond that cannot be repaired by the body, and ultimately the continuing demineralisation process results in tooth erosion and dental caries. The maintenance and repair of human tooth enamel is therefore one of the primary concerns of dentistry.

A remineralisation study using a toothpaste containing hydroxylapatite and sodium monofluorophosphate is disclosed in Hornby et al., International Dental Journal 2009, 59, 325-331. US 2007/0183984 A1 is directed to an oral composition comprising a calcium phosphate salt and a combination of acids each having a different solubility in the oral cavity, for tooth mineralisation or remineralisation.

The typical colour of enamel varies from light yellow to greyish or bluish white. Since enamel is semi-translucent, the colour of dentine and any material underneath the enamel strongly affects the appearance of a tooth. The enamel on primary teeth has a more opaque crystalline form and thus appears whiter than on permanent teeth. On radiographs, the differences in the mineralization of different portions of the tooth and surrounding periodontium can be noted; enamel appears lighter than dentin or pulp since it is denser than both and more radiopaque (cf. Bath-Balogh, Fehrenbach, "Illustrated Dental Embryology, Histology, and Anatomy", Elsevier, 2011, p. 180).

As a person ages, the adult teeth often become darker due to changes in the mineral structure of the tooth. Furthermore, the teeth can become stained by bacterial pigments, food-goods and vegetables rich with carotenoids or xanthonoids. Certain antibacterial medications like tetracycline can cause teeth stains or a reduction in the brilliance of the enamel, and ingesting coloured liquids like coffee, tea, and red wine or smoking can discolour teeth ("Tooth bleaching", Wikipedia, The Free Encyclopedia, 5 February 2014).

Methods for whitening teeth often involve a bleaching process using aggressive oxidation agents such as peroxides, and may require the entire solid composition to remain in contact with the teeth for an extended period of time. As an alternative, dentifrice compositions providing both remineralisation and whitening of teeth employing calcium salts have been suggested.

WO 2012/143220 A1 describes a composition that is suitable for remineralisation and whitening of teeth, which comprises a calcium source and regeneration-source calcium salt. A dentifrice composition comprising a water insoluble and/or slightly water-soluble calcium source and an organic acid, or its physiologically acceptable salt, is described in WO 2013/034421 A2. WO 2012/031786 A2 relates to oral care compositions with composite particle actives having a core and a coating, whereby the coating interacts with phosphate ions to produce calcium and phosphate reaction products that are suitable to adhere to tooth enamel and/or dentine to improve the characteristics of teeth.

WO 2015/150011 A1 relates to surface-reacted calcium carbonate and oral compositions containing the same for remineralisation and/or whitening of teeth. Surface-reacted calcium carbonate for use as medicament, and especially in treating dentine hypersensitivity, is described in WO 2015/140308 A1.

In view of the foregoing, there is a continuous need for compositions that are useful in the remineralisation of teeth and/or whitening of teeth.

Accordingly, it is an object of the present invention to provide a composition that is suitable to remineralise and whiten teeth and is compatible with conventional oral care ingredients. It would also be desirable to provide an oral care composition, which is gentle for use and easy to apply. It would also be desirable to provide a composition for remineralisation and/or whitening of teeth, which does not necessarily require in-office or in-surgery treatments, but can be used at home, for example, on a daily basis.

It is also an object of the present invention to provide an oral care composition which is more resistant to acid challenge. It would also be desirable to provide an oral care composition which does not necessarily needs to a have components or agents having a particle size in the nanosize range. It would also be desirable to provide an oral care composition which provides the additional benefit of being a carrier material for active agents.

The foregoing and other objects are solved by the subject-matter as defined herein in the independent claims.

According to one aspect of the present invention, an oral care composition is provided comprising

silica in an amount from 6 to 40 wt.-%, based on the total weight of the composition, and
a surface-reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and

the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277.

According to a further aspect of the present invention, an oral care composition according to any one of the preceding claims for use in remineralisation and/or whitening of teeth is provided.

Advantageous embodiments of the present invention are defined in the corresponding sub-claims.

According to one embodiment the at least one $H_3O^+$-ion donor is selected from the group consisting of hydrochloric acid, sulphuric acid, sulphurous acid, phosphoric acid, citric acid, oxalic acid, acetic acid, formic acid, and mixtures thereof, preferably the at least one $H_3O^+$-ion donor is selected from the group consisting of hydrochloric acid, sulphuric acid, sulphurous acid, phosphoric acid, oxalic acid, and mixtures thereof, and more preferably the at least one $H_3O^+$-ion donor is phosphoric acid.

According to one embodiment the surface-reacted calcium carbonate has a volume determined median particle size $d_{50}$ from 2.4 to 4.5 $\mu$m, preferably from 2.5 to 4.0 $\mu$m, and most preferably from 2.8 to 3.5 $\mu$m. According to another embodiment the surface-reacted calcium carbonate has a volume determined top cut particle size $d_{98}$ from 5 to 13 $\mu$m, preferably from 7 to 12 $\mu$m, and most preferably from 9 to 11 $\mu$m. According still another embodiment the surface-reacted calcium carbonate has a specific surface area from 60 to 107 m$^2$/g, preferably from 70 to 105 m$^2$/g, and most preferably from 90 to 100 m$^2$/g, measured using nitrogen and the BET method according to ISO 9277.

According to one embodiment the silica is present in an amount from 15 to 30 wt.-%, preferably from 15 to 25 wt.-%, and most preferably from 18 to 23 wt.-%, based on the total weight of the composition. According to another embodiment the surface-reacted calcium carbonate is present in an amount from 1 to 30 wt.-%, preferably from 2 to 15 wt.-%, more preferably from 3 to 10 wt.-%, and most preferably from 4 to 6 wt.-%, based on the total weight of the composition.

According to one embodiment the silica is present in an amount from 18 to 23 wt.-%, based on the total weight of the composition, the surface reacted calcium carbonate is present in an amount from 4 to 6 wt.-%, based on the total weight of the composition, the surface-reacted calcium carbonate is a reaction product of natural or synthetic calcium carbonate with carbon dioxide and phosphoric acid, and the surface-reacted calcium carbonate is in form of particles having a volume determined median particle size $d_{50}$ from 2.8 to 3 $\mu$m, having a volume determined top cut particle size $d_{98}$ from 9 to 11 $\mu$m, and a specific surface area from 90 to 100 m$^2$/g, measured using nitrogen and the BET method according to ISO 9277.

According to one embodiment the oral composition further comprises a fluoride compound, preferably the fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride. According to another embodiment the oral care composition further comprises an additional remineralisation and/or whitening agent, preferably selected from the group consisting of hydroxylapatite, nano-hydroxylapatite, calcium carbonate, amorphous calcium carbonate, and combinations thereof with casein phospholipids, hydrogen peroxide, carbamide peroxide, or fluoride compounds, and mixtures thereof.

According to one embodiment the oral care composition is a toothpaste, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash, preferably a chewable gum, a lozenge, a toothpaste, a toothpowder, or a mouthwash, and most preferably a toothpaste. According to another embodiment at least one active agent is associated with the surface-reacted calcium carbonate, preferably the active agent is at least one additional desensitizing agent, and more preferably the at least one additional desensitizing agent is selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof. According to still another embodiment the oral care composition has a pH between 7.5 and 10, preferably between 8 and 9.

According to one embodiment the surface-reacted calcium carbonate is obtained by a process comprising the steps of: a) providing an aqueous suspension of natural or synthetic calcium carbonate, wherein the aqueous suspension has a solids content in the range of 5 to 25 wt.-%, based on the weight of the aqueous suspension, and the amount of natural or synthetic calcium carbonate having a weight based particle size of less than 1 $\mu$m is at least 80 wt.-%, based on the total amount of natural or synthetic calcium carbonate, and b) adding at least one $H_3O^+$-ion donor to the suspension of step a), and c) treating the suspension of step a) with carbon dioxide before, during or after step b), wherein the carbon dioxide is formed in-situ by the $H_3O^+$-ion donor treatment and/or is supplied from an external source.

It should be understood that for the purpose of the present invention, the following terms have the following meaning.

For the purpose of the present invention, an "acid" is defined as Bronsted-Lowry acid, that is to say, it is an $H_3O^+$ ion provider. An "acidic salt" is defined as an $H_3O^+$ ion-provider, e.g., a hydrogen-containing salt, which is partially neutralised by an electropositive element. A "salt" is defined as an electrically neutral ionic compound formed from anions and cations. A "partially crystalline salt" is defined as a salt that, on XRD analysis, presents an essentially discrete diffraction pattern.

In accordance with the present invention, $pK_a$, is the symbol representing the acid dissociation constant associated with a given ionisable hydrogen in a given acid, and is indicative of the natural degree of dissociation of this hydrogen from this acid at equilibrium in water at a given temperature. Such $pK_a$ values may be found in reference textbooks such as Harris, D. C. "Quantitative Chemical Analysis: 3rd Edition", 1991, W.H. Freeman & Co. (USA), ISBN 0-7167-2170-8.

"Ground calcium carbonate" (GCC) in the meaning of the present invention is a calcium carbonate obtained from natural sources, such as limestone, marble, dolomite, or chalk, and processed through a wet and/or dry treatment such as grinding, screening and/or fractionating, for example, by a cyclone or classifier.

"Precipitated calcium carbonate" (PCC) in the meaning of the present invention is a synthesised material, obtained by precipitation following reaction of carbon dioxide and lime in an aqueous, semi-dry or humid environment or by precipitation of a calcium and carbonate ion source in water. PCC may be in the vateritic, calcitic or aragonitic crystal form.

For the purpose of the present invention, a "surface-reacted calcium carbonate" is a material comprising calcium carbonate and an insoluble, at least partially crystalline, non-carbonate calcium salt, preferably, extending from the surface of at least part of the calcium carbonate. The calcium ions forming said at least partially crystalline non-carbonate calcium salt originate largely from the starting calcium carbonate material that also serves to form the surface-reacted calcium carbonate core. Such salts may include $OH^-$ anions and/or crystal water.

In the meaning of the present invention "water-insoluble" materials are defined as materials which, when mixed with deionised water and filtered on a filter having a 0.2 $\mu$m pore size at 20°C to recover the liquid filtrate, provide less than or equal to 0.1 g of recovered solid material following evaporation at 95 to 100°C of 100 g of said liquid filtrate. "Water-soluble" materials are defined as materials leading to the recovery of greater than 0.1 g of recovered solid material following evaporation at 95 to 100°C of 100 g of said liquid filtrate.

Throughout the present document, the "particle size" of a calcium carbonate and other materials is described by its distribution of particle sizes. The value $d_x$ represents the diameter relative to which $x$ % by weight of the particles have diameters less than $d_x$. This means that the $d_{20}$ value is the particle size at which 20 wt.-% of all particles are smaller, and the $d_{75}$ value is the particle size at which 75 wt.-% of all particles are smaller. The $d_{50}$ value is thus the weight median particle size, i.e. 50 wt.-% of all grains are bigger and the remaining 50 wt.-% are smaller than this particle size. For the purpose of the present invention the particle size is specified as weight median particle size $d_{50}$ unless indicated otherwise. For determining the weight median particle size $d_{50}$ value a Sedigraph can be used. For the purpose of the present invention, the "particle size" of surface-reacted calcium is described as volume determined particle size distributions. For determining the volume determined particle size distribution, e.g., the volume median grain diameter ($d_{50}$) or the volume determined top cut particle size ($d_{98}$) of surface-reacted calcium carbonate, a Malvern Mastersizer 2000 can be used. The weight determined particle size distribution may correspond to the volume determined particle size if the density of all the particles is equal.

A "specific surface area (SSA)" of a calcium carbonate in the meaning of the present invention is defined as the surface area of the calcium carbonate divided by its mass. As used herein, the specific surface area is measured by nitrogen gas adsorption using the BET isotherm (ISO 9277:2010) and is specified in $m^2/g$.

An "oral care composition" in the meaning of the present invention refers to a composition suitable for the use in the mouth and for veterinary and/or human applications but especially for use in applications for the human mouth.

In the meaning of the present invention, the "radioactive dentine abrasion (RDA)" is a measure of the erosive effect of abrasives in toothpaste on tooth dentine. It involves using standardised abrasives compared against the test sample. The determination of this value is done by determining the activity while cleaning worn dentine which is radioactively marked by mild neutron irradiation. The values obtained depend on the size, quantity and surface structure of abrasive used in toothpastes. The RDA value is set by the standards DIN EN ISO 11609.

For the purpose of the present invention, the term "viscosity" or "Brookfield viscosity" refers to Brookfield viscosity. The Brookfield viscosity is for this purpose measured by a Brookfield (Type RVT) viscometer at 20°C $\pm$ 2°C at 100 rpm using an appropriate spindle and is specified in mPa·s.

A "suspension" or "slurry" in the meaning of the present invention comprises insoluble solids and water, and optionally further additives, and usually contains large amounts of solids and, thus, is more viscous and can be of higher density than the liquid from which it is formed.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

According to the present invention an oral care composition is provided. The oral care composition comprises silica in an amount from 6 to 40 wt.-%, based on the total weight of the composition, and a surface-reacted calcium carbonate in an amount from 1 to 40 wt.-%, based on the total weight of the composition. The surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277.

In the following the details and preferred embodiments of the inventive oral care composition will be set out in more details. It is to be understood that these technical details and embodiments also apply to the inventive use of said composition.

The surface-reacted calcium carbonate

According to the present invention, the oral care composition comprises a surface-reacted calcium carbonate, wherein the surface-reacted calcium carbonate is a reaction product of natural or synthetic calcium carbonate with carbon dioxide and at least one $H_3O^+$-ion donor.

Natural (or ground) calcium carbonate (GCC) is understood to be a naturally occurring form of calcium carbonate, mined from sedimentary rocks such as limestone or chalk, or from metamorphic marble rocks. Calcium carbonate is known to exist mainly as three types of crystal polymorphs: calcite, aragonite and vaterite. Calcite, the most common crystal polymorph, is considered to be the most stable crystal form of calcium carbonate. Less common is aragonite, which has a discrete or clustered needle orthorhombic crystal structure. Vaterite is the rarest calcium carbonate polymorph and is generally unstable. Natural calcium carbonate is almost exclusively of the calcitic polymorph, which is said to be trigonal-rhombohedral and represents the most stable of the calcium carbonate polymorphs. The term "source" of the calcium carbonate in the meaning of the present invention refers to the naturally occurring mineral material from which the calcium carbonate is obtained. The source of the calcium carbonate may comprise further naturally occurring components such as magnesium carbonate, aluminosilicate etc.

According to one embodiment of the present invention, the natural calcium carbonate is selected from the group consisting of marble, chalk, dolomite, limestone and mixtures thereof.

According to one embodiment of the present invention the GCC is obtained by dry grinding. According to another embodiment of the present invention the GCC is obtained by wet grinding and optionally subsequent drying.

In general, the grinding step can be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source in water or by precipitation of calcium and carbonate ions, for example $CaCl_2$ and $Na_2CO_3$, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the synthetic calcium carbonate is precipitated calcium carbonate, preferably comprising aragonitic, vateritic or calcitic mineralogical crystal forms or mixtures thereof.

According to one embodiment of the present invention, the natural or synthetic calcium carbonate is ground prior to the treatment with carbon dioxide and at least one acid. The grinding step can be carried out with any conventional grinding device such as a grinding mill known to the skilled person.

According to one embodiment of the present invention, the natural or synthetic calcium carbonate is in form of particles having a weight median particle size $d_{50}$ of equal to or less than 2 $\mu$m, preferably from 0.4 to 1.5 $\mu$m, more preferably from 0.5 to 1.0 $\mu$m, and most preferably from 0.55 to 0.9 $\mu$m, for example, 0.55 or 0.63 $\mu$m. According to a further embodiment of the present invention, the natural or synthetic calcium carbonate is in form of particles having a top cut particle size $d_{98}$ of equal to or less than 2.0 $\mu$m, preferably from 1.1 to 1.9 $\mu$m, more preferably from 1.2 to 1.8 $\mu$m, and most preferably from 1.3 to 1.7 $\mu$m, for example, 1.31 or 1.50 $\mu$m.

Preferably the surface-reacted calcium carbonate to be used in the present invention is prepared as an aqueous suspension having a pH, measured at 20°C, greater than 6.0, preferably greater than 6.5, more preferably greater than 7.0, even more preferably greater than 7.5.

In a preferred process for the preparation of the aqueous suspension of surface-reacted calcium carbonate, the natural or synthetic calcium carbonate, either finely divided, such as by grinding, or not, is suspended in water. Preferably, the slurry has a content of natural or synthetic calcium carbonate within the range of 1 wt.-% to 90 wt.-%, more preferably 3 wt.-% to 60 wt.-%, and even more preferably 5 wt.-% to 40 wt.-%, based on the weight of the slurry.

In a next step, at least one $H_3O^+$-ion donor is added to the aqueous suspension containing the natural or synthetic calcium carbonate. The at least one $H_3O^+$-ion donor can be any strong acid, medium-strong acid, or weak acid, or mixtures thereof, generating $H_3O^+$ ions under the preparation conditions. According to the present invention, the at least one $H_3O^+$-ion donor can also be an acidic salt, generating $H_3O^+$ ions under the preparation conditions.

According to one embodiment, the at least one $H_3O^+$-ion donor is a strong acid having a $pK_a$ of 0 or less at 20°C. According to another embodiment, the at least one $H_3O^+$-ion donor is a medium-strong acid having a $pK_a$ value from 0 to 2.5 at 20°C. If the $pK_a$ at 20°C is 0 or less, the $H_3O^+$-ion donor is preferably selected from sulphuric acid, hydrochloric acid, or mixtures thereof. If the $pK_a$ at 20°C is from 0 to 2.5, the $H_3O^+$-ion donor is preferably selected from $H_2SO_3$, $H_3PO_4$, oxalic acid, or mixtures thereof. The at least one $H_3O^+$-ion donor can also be an acidic salt, for example, $HSO_4^-$ or $H_2PO_4^-$, being at least partially neutralized by a corresponding cation such as $Li^+$, $Na^+$ or $K^+$, or $HPO_4^{2-}$, being at least partially neutralised by a corresponding cation such as $Li^+$, $Na^+$, $K^+$, $Mg^{2+}$ or $Ca^{2+}$. The at least one $H_3O^+$-ion donor can also be a mixture of one or more acids and one or more acidic salts.

According to still another embodiment, the at least one $H_3O^+$-ion donor is a weak acid having a $pK_a$ value of greater than 2.5 and less than or equal to 7, when measured at 20°C, associated with the ionisation of the first available hydrogen, and having a corresponding anion formed on loss of this first available hydrogen, which is capable of forming water-soluble calcium salts. According to a preferred embodiment, the weak acid has a $pK_a$ value from 2.6 to 5 at 20°C, and more preferably the weak acid is selected from the group consisting of acetic acid, formic acid, propanoic acid, and mixtures thereof.

In case a weak acid is used, after addition of said acid to the aqueous suspension containing the natural or synthetic calcium carbonate, at least one water-soluble salt, which in the case of a hydrogen-containing salt has a $pK_a$ of greater than 7, when measured at 20°C, associated with the ionisation of the first available hydrogen, and the salt anion of which is capable of forming water-insoluble calcium salts, is additionally added. The cation of said water-soluble salt is preferably selected from the group consisting of potassium, sodium, lithium and mixtures thereof. In a more preferred embodiment, said cation is sodium. It is of note that depending on the charge of the anion, more than one of said cations may be present to provide an electrically neutral ionic compound. The anion of said water-soluble salt is preferably selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one $H_3O^+$-ion donor is selected from the group consisting of hydrochloric acid, sulphuric acid, sulphurous acid, phosphoric acid, citric acid, oxalic acid, acetic acid, formic acid, and mixtures thereof. Preferably the at least one $H_3O^+$-ion donor is selected from the group consisting of hydrochloric acid, sulphuric acid, sulphurous acid, phosphoric acid, oxalic acid, $H_2PO_4^-$, being at least partially neutralised by a corresponding cation such as $Li^+$, $Na^+$ or $K^+$, $HPO_4^{2-}$, being at least partially neutralised by a corresponding cation such as $Li^+$, $Na^+$, $K^+$, $Mg^{2+}$ or $Ca^{2+}$, and mixtures thereof, more preferably the at least one $H_3O^+$-ion donor is selected from the group consisting of hydrochloric acid, sulphuric acid, sulphurous acid, phosphoric acid, oxalic acid, or mixtures thereof, and most preferably, the at least one $H_3O^+$-ion donor is phosphoric acid. Without being bound to any theory, the inventors believe that the use of phosphoric acid can be beneficial in remineralisation and/or whitening of teeth.

The at least one $H_3O^+$-ion donor can be added to the suspension as a concentrated solution or a more diluted solution. Preferably, the molar ratio of the at least one $H_3O^+$-ion donor to the natural or synthetic calcium carbonate is from 0.05 to 4, more preferably from 0.1 to 2.

As an alternative, it is also possible to add the at least one $H_3O^+$-ion donor to the water before the natural or synthetic calcium carbonate is suspended.

According to the present invention, the surface-reacted calcium carbonate is obtained by treating the natural or synthetic calcium carbonate with carbon dioxide. The carbon dioxide can be formed in situ by the acid treatment and/or can be supplied from an external source. If a strong acid such as sulphuric acid or hydrochloric acid or medium-strong acid such as phosphoric acid is used for the $H_3O^+$-ion donor treatment of the natural or synthetic calcium carbonate, the carbon dioxide is automatically formed. Alternatively or additionally, the carbon dioxide can be supplied from an external source.

According to one embodiment, the surface-reacted calcium carbonate is a reaction product of natural or synthetic calcium carbonate with carbon dioxide and at least one $H_3O^+$-ion donor, wherein the carbon dioxide is formed in situ as a result of contacting the at least one acid with the natural or synthetic calcium carbonate and/or is supplied from an external source.

$H_3O^+$-ion donor treatment and treatment with carbon dioxide can be carried out simultaneously which is the case when a strong or medium-strong acid is used. It is also possible to carry out acid treatment first, e.g. with a medium strong acid having a $pK_a$ in the range of 0 to 2.5 at 20°C, wherein carbon dioxide is formed in situ, and thus, the carbon dioxide treatment will automatically be carried out simultaneously with the acid treatment, followed by the additional treatment with carbon dioxide supplied from an external source.

Preferably, the concentration of gaseous carbon dioxide in the suspension is, in terms of volume, such that the ratio (volume of suspension): (volume of gaseous $CO_2$) is from 1:0.05 to 1:20, even more preferably from 1:0.05 to 1:5.

In a preferred embodiment, the $H_3O^+$-ion donor treatment step and/or the carbon dioxide treatment step are repeated at least once, more preferably several times. According to one embodiment, the at least one $H_3O^+$-ion donor is added over a time period of at least 30 min, preferably at least 45 min, and more preferably at least 1 h.

Subsequent to the $H_3O^+$-ion donor treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted calcium carbonate as an aqueous suspension having a pH of greater than 6.0, preferably greater than 6.5, more preferably greater than 7.0, even more preferably greater than 7.5. If the aqueous suspension is allowed to reach equilibrium, the pH is greater than 7. A pH of greater than 6.0 can be adjusted without the addition of a base when stirring of the aqueous suspension is continued for a sufficient time period, preferably 1 hour to 10 hours, more preferably 1 to 5 hours.

Alternatively, prior to reaching equilibrium, which occurs at a pH greater than 7, the pH of the aqueous suspension may be increased to a value greater than 6 by adding a base subsequent to carbon dioxide treatment. Any conventional base such as sodium hydroxide or potassium hydroxide can be used.

Further details about the preparation of the surface-reacted natural calcium carbonate are disclosed in WO 00/39222 A1 and US 2004/0020410 A1, wherein the surface-reacted natural calcium carbonate is described as a filler for paper manufacture. The preparation of surface-reacted calcium carbonate with weak acids is disclosed in EP 2 264 108 A1. The preparation of surface-reacted calcium carbonate and its use in purification processes is disclosed in EP 1 974 806 A1, EP 1 982 759 A1, and EP 1 974 807 A1. The use of surface-reacted calcium carbonate as carrier for the controlled release of active agents is described in WO 2010/037753 A1.

Similarly, surface-reacted precipitated calcium carbonate is obtained. As can be taken in detail from EP 2 070 991 A1, surface-reacted precipitated calcium carbonate is obtained by contacting precipitated calcium carbonate with $H_3O^+$ ions and with anions being solubilised in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilised calcium ions correspond to an excess of solubilised calcium ions relative to the solubilised calcium ions naturally generated on dissolution of precipitated calcium carbonate by $H_3O^+$ ions, where said $H_3O^+$ ions are provided solely in the form of a counterion to the anion, i.e. via the addition of the anion in the form of an acid or non-calcium acid salt, and in absence of any further calcium ion or calcium ion generating source.

Said excess solubilised calcium ions are preferably provided by the addition of a soluble neutral or acid calcium salt, or by the addition of an acid or a neutral or acid non-calcium salt which generates a soluble neutral or acid calcium salt in situ.

Said $H_3O^+$ ions may be provided by the addition of an acid or an acid salt of said anion, or the addition of an acid or an acid salt which simultaneously serves to provide all or part of said excess solubilised calcium ions.

According to one embodiment of the present invention, the surface-reacted calcium carbonate is obtained by a process comprising the steps of:

a) providing an aqueous suspension of natural or synthetic calcium carbonate, wherein the aqueous suspension has a solids content in the range of 5 to 25 wt.-%, based on the weight of the aqueous suspension, and the amount of natural or synthetic calcium carbonate having a weight based particle size of less than 1 $\mu$m is at least 80 wt.-%, based on the total amount of natural or synthetic calcium carbonate, and

b) adding at least one $H_3O^+$-ion donor to the suspension of step a), and

c) treating the suspension of step a) with carbon dioxide before, during or after step b), wherein the carbon dioxide is formed in-situ by the $H_3O^+$-ion donor treatment and/or is supplied from an external source.

According to a preferred embodiment, the amount of natural or synthetic calcium carbonate having a weight based particle size of less than 1 $\mu$m is at least 90 wt.-%, based on the total amount of natural or synthetic calcium carbonate.

The surface-reacted calcium carbonate can be kept in suspension, optionally further stabilised by a dispersant. Conventional dispersants known to the skilled person can be used. A preferred dispersant is polyacrylic acid and/or carboxymethylcellulose.

Alternatively, the aqueous suspension described above can be dried, thereby obtaining the solid (i.e. dry or containing as little water that it is not in a fluid form) surface-reacted natural or synthetic calcium carbonate in the form of granules or a powder.

According to the present invention, the surface-reacted calcium carbonate is in form of particles having a weight median particle size $d_{50}$ from 2 to 5 $\mu$m. According to one embodiment, the surface-reacted calcium carbonate has a volume determined median particle size $d_{50}$ from 2.4 to 4.5 $\mu$m, preferably from 2.5 to 4.0 $\mu$m, and most preferably from 2.8 to 3.5 $\mu$m. The volume determined median particle size ($d_{50}$) can be determined by laser diffraction measurements, for example, by using a Malvern Mastersizer 2000.

According to the present invention, the surface-reacted calcium carbonate is in form of particles having a top cut particle size $d_{98}$ from 4 to 15 $\mu$m. According to one embodiment, the surface-reacted calcium carbonate has a volume determined top cut particle size $d_{98}$ from 5 to 13 $\mu$m, preferably from 7 to 12 $\mu$m, and most preferably from 9 to 11 $\mu$m. The volume determined top cut particle size ($d_{98}$) can be determined by laser diffraction measurements, for example, by using a Malvern Mastersizer 2000.

According to one embodiment, the surface-reacted calcium carbonate has a volume determined median particle size $d_{50}$ from 2.4 to 4.5 $\mu$m, preferably from 2.5 to 4.0 $\mu$m, and most preferably from 2.8 to 3.5 $\mu$m, and a volume determined top cut particle size $d_{98}$ from 5 to 13 $\mu$m, preferably from 7 to 12 $\mu$m, and most preferably from 9 to 11 $\mu$m. The volume determined median particle size ($d_{50}$) and volume determined top cut particle size ($d_{98}$) can be determined by laser diffraction measurements, for example, by using a Malvern Mastersizer 2000.

According to the present invention, the surface-reacted calcium carbonate has a specific surface area of from 5 m$^2$/g to 110 m$^2$/g, measured using nitrogen and the BET method according to ISO 9277. According to one embodiment, the surface-reacted calcium carbonate has a specific surface area from 60 to 107 m$^2$/g, preferably from 70 to 105 m$^2$/g, and most preferably from 90 to 100 m$^2$/g, measured using nitrogen and the BET method according to ISO 9277.

According to one embodiment of the present invention, the surface-reacted calcium carbonate comprises an insoluble, at least partially crystalline calcium salt of an anion of the at least one acid, which is formed on the surface of the natural or synthetic calcium carbonate. According to one embodiment, the insoluble, at least partially crystalline salt of an anion of the at least one acid covers the surface of the natural or synthetic calcium carbonate at least partially, preferably completely. Depending on the employed at least one acid, the anion may be sulphate, sulphite, phosphate, citrate, oxalate, acetate, formiate and/or chloride.

According to one preferred embodiment, the surface-reacted calcium carbonate is a reaction product of natural calcium carbonate and at least one $H_3O^+$-ion donor, preferably phosphoric acid.

The surface-reacted calcium carbonate has a good loading capacity and can be used as a carrier in oral care. For example, the surface-reacted calcium carbonate is capable of associating and transporting an active agent. The association preferably is an adsorption onto the surface of the surface-reacted calcium carbonate particles, be it the outer or the inner surface of the particles or an absorption into the particles, which is possible due to their porosity.

In this respect, it is believed that because of the intra and interpore structure of the surface-reacted calcium carbonate, this material is a superior agent to deliver previously ad/absorbed materials over time relative to common materials having similar specific surface areas.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m (~nm). The equilibration time used at each pressure step is 20 seconds. The sample material is sealed in a 5 cm$^3$ chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine interparticle packing of the particles themselves. If they also have intraparticle pores, then this region appears bi modal, and by taking the specific pore volume intruded by mercury into pores finer than the modal turning point, i.e. finer than the bi-modal point of inflection, define the specific intraparticle pore volume. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

Preferably, the surface-reacted calcium carbonate has an intra-particle intruded specific pore volume in the range from 0.1 to 2.3 cm$^3$/g, more preferably from 0.2 to 2.0 cm$^3$/g, especially preferably from 0.4 to 1.8 cm$^3$/g and most preferably from 0.6 to 1.6 cm$^3$/g, calculated from mercury porosimetry measurement.

Thus, generally, any agent fitting into the intra- and/or inter particle pores of the surface-reacted calcium carbonate is suitable to be transported by the surface- reacted calcium carbonate according to the invention. For example, active agents such as those selected from the group comprising pharmaceutically active agents, biologically active agents, disinfecting agents, preservatives such as triclosan, flavouring agents, surfactants like defoamers, or additional desensitizing agents can be used. According to one embodiment, at least one active agent is associated with the surface-reacted calcium carbonate. According to a preferred embodiment the active agent is at least one additional desensitising agent, preferably selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof. Hydroxylapatite, also called hydroxyapatite, is a naturally occurring mineral form of calcium apatite with the formula $Ca_5(PO_4)_3(OH)$. According to an exemplary embodiment, the hydroxylapatite is a nanosized hydroxylapatite, also called nano-hydoxylapatite.

The oral care composition

According to the present invention, the oral care composition comprises silica in an amount from 6 to 40 wt.-%, based on the total weight of the composition, and a surface reacted calcium carbonate in an amount from 1 to 40 wt.-%, based on the total weight of the composition.

The inventors of the present invention surprisingly found that the combination of silica with a surface-reacted calcium carbonate having the specific characteristics defined in claim 1 is especially effective in remineralisation and/or whitening of teeth. For example, it was found by the inventors that the inventive oral care composition comprising a combination of silica with a surface-reacted calcium carbonate can be just as good, or even more effective in remineralisation of teeth as oral care compositions of the prior art comprising fluoride, microcrystalline or nanocrystalline hydroxylapatite.

Furthermore, surface-reacted calcium carbonate differs from conventional calcium carbonate in several aspects. For example, it is porous, has a low density, a low abrasivity and can be easily formulated into a toothpaste. Furthermore, unlike conventional calcium carbonate, surface-reacted calcium carbonate comprises a porous, platy or lamellar surface structure. Without being bound to any theory, it is believed that during application of the surface-reacted calcium carbonate, for example, on the tooth of a patient, the surface-reacted calcium carbonate breaks into pieces, whereby the porous platy or lamellar surface structure elements are cleaved from the surface of the surface-reacted calcium carbonate. Regarding a study of the compressibility and compactibility of surface-reacted calcium carbonate, reference is made to Stirnimann et al., International Journal of Pharmaceutics 466 (2014) 266-275. Said cleaved porous platy or lamellar surface structure elements may provide an improved adherence to the teeth enamel. The surface treatment also renders the surface-reacted calcium carbonate more resistant against acids. Therefore, the surface-reacted calcium carbonate may be more stable under acidic conditions, for example, during consumption of acidic beverages such as soft drinks or acidic dishes such as salads with vinegar-based dressings. Another advantage of the surface-reacted calcium carbonate is that it can be used in a micrometre particle size range, and thus, the use of nanosized particles can be avoided.

According to one embodiment of the present invention, the silica is present in an amount from 15 to 30 wt.-%, preferably from 15 to 25 wt.-%, and most preferably from 18 to 23 wt.-%, based on the total weight of the composition. As used herein, the term "silica" refers to silicon dioxide materials. Silica materials which are suitable for oral application are known to the skilled person. According to one embodiment, the silica is selected from the group consisting of hydrated silica, colloidal silica, abrasive silica, cleaning silica, and mixtures thereof. According to a preferred embodiment of the present invention, the silica has a particle size in the micrometer range, for example, from 1 to 100 $\mu$m, preferably from 2 to 50 $\mu$m, more preferably from 2.5 to 20 $\mu$m.

According to one embodiment, the silica has a radioactive dentine abrasion (RDA) value of less than 250, preferably less than 200, and more preferably less than 180. According to another embodiment of the present invention, the oral care composition is a toothpaste for sensitive teeth and/or for children's teeth, and preferably the silica has an RDA of less than 50, and most preferably less than 35. According to one embodiment, the surface-reacted calcium carbonate has a radioactive dentine abrasion (RDA) value of less than 70, preferably less than 50, and more preferably less than 35. According to one embodiment of the present invention, the oral care composition is a toothpaste for sensitive teeth and/or for children's teeth, and preferably the surface-reacted calcium carbonate has an RDA of less than 50, and most preferably less than 35. According to another embodiment of the present invention, the oral care composition is a toothpaste for sensitive teeth and/or for children's teeth, and the silica has an RDA of less than 50, and most preferably less than 35, and the surface-reacted calcium carbonate has an RDA of less than 50, and most preferably less than 35.

According to one embodiment of the present invention, the surface-reacted calcium carbonate is present in an amount from 1 to 30 wt.-%, preferably from 2 to 15 wt.-%, more preferably from 3 to 10 wt.-%, and most preferably from 4 to 6 wt.-%, based on the total weight of the composition.

The surface-reacted calcium carbonate can consist of only one type of surface-reacted calcium carbonate or can be a mixture of two or more types of surface-reacted calcium carbonate. The oral care composition of the present invention may contain the surface-reacted calcium carbonate as the only remineralisation and/or whitening agent. Alternatively, the oral care composition of the present invention may contain the surface-reacted calcium carbonate in combination with at least one additional remineralisation and/or whitening agent.

According to one embodiment, the oral care composition comprises at least one additional remineralisation agent. Preferably, the additional remineralisation agent is selected from the group consisting of hydroxylapatite, e.g. nano-hydroxylapatite, calcium carbonate, e.g. amorphous calcium carbonate, and combinations thereof with casein phospholipids, or fluoride compounds, and mixtures thereof. Amorphous calcium carbonate is an amorphous and least stable polymorph of calcium carbonate and aside from several specialized organisms it is not found naturally.

According to another embodiment, the oral care composition comprises at least one additional whitening agent. The additional whitening agent can be a bleaching agent, an abrasive, or a remineralisation agent, and is preferably selected from the group consisting of hydrogen peroxide, carbamide peroxide, hydroxylapatite, calcium carbonate, and mixtures thereof.

According to one embodiment of the present invention, the at least one additional remineralisation and/or whitening agent is selected from the group consisting of hydroxylapatite, e.g. nano-hydroxylapatite, calcium carbonate, e.g. amorphous calcium carbonate, and combinations thereof with casein phospholipids, hydrogen peroxide, carbamide peroxide, fluoride compounds, and mixtures thereof.

According to one embodiment, the additional remineralisation and/or whitening agent has a weight median particle size $d_{50}$ from 10 nm to 100 $\mu$m, preferably from 0.1 to 50 $\mu$m, more preferably from 1 to 20 $\mu$m, and most preferably from 2 to 10 $\mu$m.

The at least one additional remineralisation and/or whitening agent can be present in the oral care composition in an amount from 1 to 20 wt.-%, preferably from 1.5 to 15 wt.-%, more preferably from 2 to 10 wt.-%, based on the total weight of the composition.

According to one embodiment, the oral care composition of the present invention comprises from 1 to 40 wt.-% of the surface-reacted calcium carbonate and from 1 to 20 wt.-% of an additional remineralisation and/or whitening agent, based on the total weight of the composition.

The oral care composition of the present invention can be, for example, a toothpaste, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash. According to one embodiment of the present invention, the oral care composition is a chewable gum, a lozenge, a toothpaste, a toothpowder, or a mouthwash, and preferably a toothpaste.

According to a preferred embodiment of the present invention, the oral care composition comprises silica in an amount from 18 to 23 wt.-%, based on the total weight of the composition, and surface-reacted calcium carbonate in an amount from 4 to 6 wt.-%, based on the total weight of the composition, wherein the surface-reacted calcium carbonate is a reaction product of natural or synthetic calcium carbonate with carbon dioxide and phosphoric acid, and the surface-reacted calcium carbonate is in form of particles having a volume determined median particle size $d_{50}$ from 2.8 to 3 $\mu$m, having a volume determined top cut particle size $d_{98}$ from 9 to 11 $\mu$m, and a specific surface area from 90 to 100 m$^2$/g, measured using nitrogen and the BET method according to ISO 9277. Preferably, the oral care composition is a chewable gum, a lozenge, a toothpaste, a toothpowder, or a mouthwash, and the surface-reacted calcium carbonate is a reaction product of natural or synthetic calcium carbonate with carbon dioxide and phosphoric acid.

According to one embodiment of the present invention, the oral care composition has a pH between 7.5 and 10, preferably between 8 and 9.

The inventive oral care composition can be used in combination with a fluoride compound. The inventors surprisingly found that adding a fluoride compound to the inventive oral composition may further improve remineralisation and/or whitening of teeth.

According to a preferred embodiment, the oral composition further comprises a fluoride compound. The fluoride compound can be selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof. Preferably, the fluoride compound is sodium monofluorophosphate and/or sodium fluoride. Good results can be achieved by employing an amount of fluoride compound to provide available fluoride ion in the range of 300 to 2 000 ppm in the oral care composition, preferably about 1 450 ppm.

According to one embodiment, an oral care composition, preferably a chewable gum, a lozenge, a toothpaste, a toothpowder, or a mouthwash, is provided, comprising a silica in an amount from 6 to 40 wt.-%, based on the total weight of the composition, and a surface reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277, wherein the oral composition further comprises a fluoride compound, preferably selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably selected from sodium monofluorophosphate and/or sodium fluoride.

In addition to the surface-reacted calcium carbonate, the optional additional remineralisation and/or whitening agent, and the optional fluoride compound, the oral care composition may further comprise bioadhesive polymers, surfactants, binders, humectants, desensitising agents, flavouring agents, sweetening agents and/or water.

According to one embodiment of the present invention, the oral care composition comprises a bioadhesive polymer. The bioadhesive polymer may include any polymer that promotes adhesion of the surface-reacted calcium carbonate to teeth or tooth surface and remains on the teeth or tooth surface for an extended period of time, for example, 1 hour, 3 hours, 5 hours, 10 hours or 24 hours. In certain embodiments, the bioadhesive polymer may become more adhesive when the oral care composition is moistened with, for example, water or saliva. In other embodiments, the bioadhesive polymer is a material or combination of materials that enhance the retention of the active ingredient on the teeth or a tooth surface onto which the composition is applied. Such bioadhesive polymers include, for example, hydrophilic organic polymers, hydrophobic organic polymers, silicone gums, silicas, and combinations thereof. According to one embodiment, the bioadhesive polymer is selected from the group consisting of hydroxyethyl methacrylate, PEG/PPG copolymers, polyvinylmethylether/maleic anhydride copolymers, polyvinylpyrrolidone (PVP), cross-linked PVP, shellac, polyethylene oxide, methacrylates, acrylates copolymers, methacrylic copolymers, vinylpyrrolidone/vinyl acetate copolymers, polyvinyl caprolactum, polylactides, silicone resins, silicone adhesives, chitosan, milk proteins (casein), amelogenin, ester gum, and combinations thereof.

Suitable surfactants are generally anionic organic synthetic surfactants throughout a wide pH range. Representative of such surfactants used in the range of about 0.5 to 5 wt.-%, based on the total weight of the oral care composition, are water-soluble salts of $C_{10}$-$C_{18}$ alkyl sulphates, such as sodium lauryl sulphate, of sulphonated monoglycerides of fatty acids, such as sodium monoglyceride sulphonates, of fatty acid amides of taurine, such as sodium N-methyl-N-palmitoyltauride, and of fatty acid esters of isethionic acid, and aliphatic acylamides, such as sodium N-lauroyl sarcosinate. However, surfactants obtained from natural sources such as cocamidopropyl betaine may also be used.

Suitable binders or thickening agents to provide the desired consistency are, for example, hydroxyethylcellulose, sodium carboxymethylcellulose, natural gums, such as gum karaya, gum arabic, gum tragacanth, xanthan gum or cellulose gum. Generally, from 0.5 to 5 wt.-%, based on the total weight of the oral care composition, can be used.

Desensitising agents can be selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof.

Various humectants known to the skilled person can be used, such as glycerine, sorbitol and other polyhydric alcohols, for example, in an amount from 20 to 40 wt.-%, based on the total weight of the oral care composition. Examples of suitable flavouring agents include oil of wintergreen, oil of spearmint, oil of peppermint, oil of clove, oil of sassafras and the like. Saccharin, aspartame, dextrose, or levulose can be used as sweetening agents, for example, in an amount from 0.01 to 1 wt.-%, based on the total weight of the oral care composition. Preservatives such as sodium benzoate may be present in an amount from 0.01 to 1 wt.-%, based on the total weight of the oral care composition. Colorants such as titanium dioxide may also be added to the oral care composition, for example, in an amount from 0.01 to 1.5 wt.-%, based on the total weight of the oral care composition.

The oral care composition of the present invention may also contain a material selected from the group consisting of alumina, aluminosilicate, metaphosphate, tricalcium phosphate, calcium pyrophosphate, ground calcium carbonate, precipitated calcium carbonate, sodium bicarbonate, bentonite, kaolin, aluminium hydroxide, calcium hydrogen phosphate, hydroxylapatite, and mixtures thereof. Said material may be present in an amount from 1 to 40 wt.-%, based on the total weight of the oral care composition. According to one embodiment, the oral care composition contains a material being selected from the group consisting of ground calcium carbonate, precipitated calcium carbonate, aluminium hydroxide, calcium hydrogen phosphate, silica, hydroxylapatite, and mixtures thereof. According to a preferred embodiment of the present invention, the oral care composition comprises surface-reacted calcium carbonate, wherein the surface-reacted calcium carbonate is a reaction product of natural or synthetic calcium carbonate with carbon dioxide and at least one acid, and calcium carbonate, preferably ground calcium carbonate and/or precipitated calcium carbonate.

According to one embodiment of the present invention, the oral care composition is a tooth paste. The toothpaste may be produced by a method comprising the following steps:

I) providing a mixture of water and a humectants, and optionally at least one of a thickener, a preservative, a fluoride, and a sweetener,

II) adding silica in an amount from 6 to 40 wt.-%, based on the total weight of the composition, a surface-reacted calcium carbonate in an amount from 1 to 40 wt.-%, based on the total weight of the composition, and optionally a colorant, to the mixture of step I), wherein the surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277,

III) adding a surfactant to the mixture of step II), and

IV) optionally, adding a flavouring agent to the mixture of step III).

However, a toothpaste of the present invention may also be produced by any other method known to the skilled person.

Therapeutic and cosmetic use

It was found that an oral care composition according to the present invention can be used in remineralisation and/or whitening of teeth.

According to one embodiment of the present invention, an oral care composition for use in remineralisation of teeth is provided, wherein the oral care composition comprises silica in an amount from 6 to 40 wt.-%, based on the total weight of the composition, and a surface-reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277.

According to another embodiment of the present invention, an oral care composition for use in whitening of teeth enamel is provided, wherein the oral care composition comprises silica in an amount from 6 to 40 wt.-%, based on the total weight of the composition, and a surface-reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277.

According to another embodiment of the present invention, an oral care composition for use in remineralisation and whitening of teeth enamel is provided, wherein the oral care composition comprises silica in an amount from 6 to 40 wt.-%, based on the total weight of the composition, and a surface-reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 $m^2/g$, measured using nitrogen and the BET method according to ISO 9277.

It was surprisingly found by the inventors that the oral care composition may be also useful for smoothening of teeth surfaces. Without being bound to any theory, it is believed that the cleaved porous platy or lamellar surface structure elements, generated by the breakage of the surface-reacted calcium carbonate, adhere to the enamel surface and seal surface defects, and thus, render the enamel surface more smooth. It is further believed that the smoother surface may prevent or reduce the adherence of bacteria and stains, which in turn may reduce the risk of bad breath and tooth decay.

According to one further aspect, an oral care composition for use in smoothing of a teeth surface is provided, wherein the oral care composition comprises silica in an amount from 6 to 40 wt.-%, based on the total weight of the composition, and a surface-reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 $m^2/g$, measured using nitrogen and the BET method according to ISO 9277.

The oral care composition of the present invention may be used in professional, in-office treatment or in at home treatment.

According to one embodiment, the oral care composition for use in remineralisation and/whitening of teeth is used in a method comprising the step of administering to at least one tooth of a patient a therapeutically effective amount of the surface-reacted calcium carbonate at least once a day, preferably twice a day and more preferably three-times a day. A "therapeutically effective" amount of the surface-reacted calcium carbonate is an amount that is sufficient to have the desired therapeutic or prophylactic effect in the human subject to whom the active agent is administered, without undue adverse side effects (such as toxicity, irritation, or allergic response), commensurate with a reasonable benefit/risk ratio when used in the manner of this invention. The specific effective amount will vary with such factors as the particular condition being treated, the physical condition of the subject, the nature of concurrent therapy (if any), the specific dosage form, the oral care composition employed, and the desired dosage regimen.

According to one embodiment, the oral composition for use in remineralisation and/or whitening of teeth is used in a method comprising the step of applying the composition to at least one tooth of a patient for an effective amount of time, preferably the composition remains on the at least one tooth for at least 1 min, at least 15 min, at least 30 min, at least 1 hour, at least 2 hours, at least 12 hours or at least 24 hours.

The oral care composition of the present invention may be effective for whitening of teeth even in the absence of any oxidative whitening compound. According to a preferred embodiment of the present invention, the oral care composition does not contain an oxidative whitening compound.

According to one embodiment, the oral composition of the present invention is used in a cosmetic method for whitening teeth, comprising the step of applying the composition to at least one tooth of an individual for an effective amount of time, preferably the composition remains on the at least one tooth for at least 1 min, at least 15 min, at least 30 min, at least 1 hour, at least 2 hours, at least 12 hours or at least 24 hours.

The scope and interest of the present invention will be better understood based on the following figure and examples which are intended to illustrate certain embodiments of the present invention and are non-limitative.

Description of the figure:

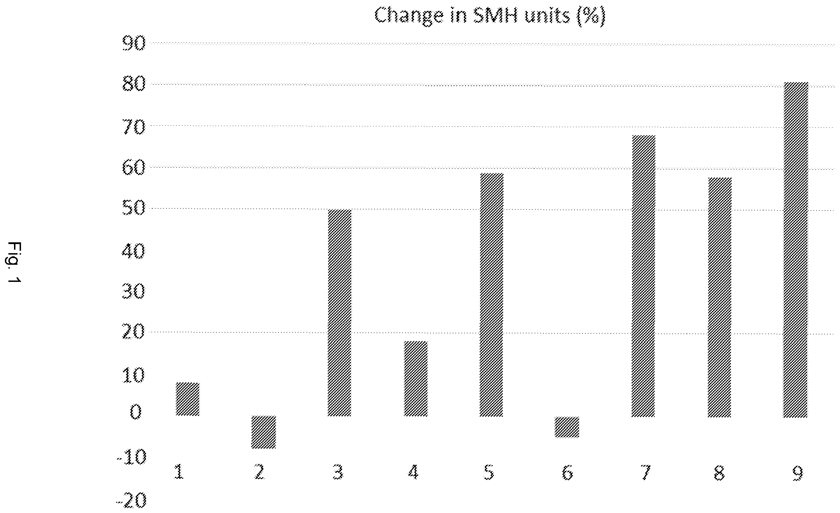

Fig. 1 shows a plot of the results of the surface micro hardness (SMH) measurements for the toothpaste samples prepared according to Example 1.

**Examples**

**1. Measurement methods**

In the following, measurement methods implemented in the examples are described.

Particle size distribution

Volume determined median particle size $d_{50}$(vol) and the volume determined top cut particle size $d_{98}$(vol) was evaluated using a Malvern Mastersizer 2000 Laser Diffraction System (Malvern Instruments Plc., Great Britain). The $d_{50}$(vol) or $d_{98}$(vol) value indicates a diameter value such that 50 % or 98 % by volume, respectively, of the particles have a diameter of less than this value. The raw data obtained by the measurement was analyzed using the Mie theory, with a particle refractive index of 1.57 and an absorption index of 0.005. The methods and instruments are known to the skilled person and are commonly used to determine particle size distributions of fillers and pigments.

The weight determined median particle size $d_{50}$(wt) was measured by the sedimentation method, which is an analysis of sedimentation behaviour in a gravimetric field. The measurement was made with a Sedigraph™ 5100 or 5120 of Micromeritics Instrument Corporation, USA. The method and the instrument are known to the skilled person and are commonly used to determine particle size distributions of fillers and pigments. The measurement was carried out in an aqueous solution of 0.1 wt.-% $Na_4P_2O_7$. The samples were dispersed using a high speed stirrer and supersonicated.

Specific surface area (SSA)

The specific surface area was measured via the BET method according to ISO 9277 using nitrogen, following conditioning of the sample by heating at 250°C for a period of 30 minutes. Prior to such measurements, the sample was filtered within a Büchner funnel, rinsed with deionised water and dried overnight at 90 to 100°C in an oven. Subsequently, the dry cake was ground thoroughly in a mortar and the resulting powder was placed in a moisture balance at 130°C until a constant weight was reached.

Intra-particle intruded specific pore volume (in $cm^3$/g)

The specific pore volume was measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m (~ nm). The equilibration time used at each pressure step was 20 seconds. The sample material was sealed in a 5 $cm^3$ chamber powder penetrometer for analysis. The data were corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine inter-particle packing of the particles themselves. If they also have intra-particle pores, then this region appears bi-modal, and by taking the specific pore volume intruded by mercury into pores finer than the modal turning point, i.e. finer than the bi-modal point of inflection, the specific intra-particle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the inter-particle pore region and the intra-particle pore region, if present. Knowing the intra-particle pore diameter range it is possible to subtract the remainder inter-particle and inter-agglomerate pore volume from the total pore volume to deliver the desired pore volume of the internal pores alone in terms of the pore volume per unit mass (specific pore volume). The same principle of subtraction, of course, applies for isolating any of the other pore size regions of interest.

Surface microhardness (SHM) measurements

The surface micro hardness (SMH) values were determined using a MicroMet 5103 Hardness testing machine with a Knoop indenter and the MicroMet MHT Software (Buehler Ltd., USA) with a 50 g load, 10 s indent time and 5 indents per block.

**2. Pigment materials (Ingredient B)**

Surface-reacted calcium carbonate 1 (SRCC 1)

SRCC 1 had a $d_{50}$(vol) = 2.9 $\mu$m, $d_{98}$(vol) = 9.8 $\mu$m, SSA = 94.5 m$^2$/g with an intra-particle intruded specific pore volume of 0.723 cm$^3$/g (for the pore diameter range of 0.004 to 0.18 $\mu$m).

SRCC 1 was obtained by preparing 10 litres of an aqueous suspension of ground calcium carbonate in a mixing vessel by adjusting the solids content of a ground limestone calcium carbonate from Omya SAS, Orgon, having a mass based median particle size distribution of 90% less than 1 $\mu$m, as determined by sedimentation, such that a solids content of 10 wt.-%, based on the total weight of the aqueous suspension, is obtained.

Whilst mixing the slurry, 2.7 kg phosphoric acid was added in form of an aqueous solution containing 20 wt.-% phosphoric acid to said suspension over a period of 44 minutes at a temperature of 70°C. After the addition of the acid, the slurry was stirred for additional 5 minutes, before removing it from the vessel and drying.

Surface-reacted calcium carbonate 2 (SRCC 2)

SRCC 2 had a $d_{50}$(vol) = 9.3 $\mu$m, $d_{98}$(vol) = 23.5 $\mu$m, SSA = 39.3 m$^2$/g with an intra-particle intruded specific pore volume of 0.83 cm$^3$/g (for the pore diameter range of 0.004 to 0.517 $\mu$m).

SRCC 2 was obtained by preparing 10 litres of an aqueous suspension of ground calcium carbonate in a mixing vessel by adjusting the solids content of a ground limestone calcium carbonate from Omya SAS, Orgon having a mass based median particle size of 3.0 $\mu$m, as determined by sedimentation, such that a solids content of 15 wt.-%, based on the total weight of the aqueous suspension, is obtained.

Whilst mixing the slurry, 2.8 kg phosphoric acid was added in form of an aqueous solution containing 30 wt.-% phosphoric acid to said suspension over a period of 10 minutes at a temperature of 70°C. After the addition of the acid, the slurry was stirred for additional 5 minutes, before removing it from the vessel and drying.

Nano-hydroxyapatite (Nano-HAP)

NanoXIM ($d_{50}$ < 50 nm), commercially available from Fluidinova, Portugal, as a highly dispersed aqueous paste.

Micro-hydroxyapatite (Micro-HAP)

Budenheim C73-08 ($d_{50}$ = 4.27 $\mu$m), commercially available from Chemische Fabrik Budenheim KG, Germany.

Silica

Sorbosil AC35 ($d_{50}$ = 10.10 $\mu$m), commercially available from PQ Corporation, UK.

Titanium dioxide

Titanium dioxide ($d_{50}$ = 0.45 $\mu$m), commercially available from Sigma-Aldrich, Switzerland. Titanium dioxide is neither an abrasive nor added for remineralisation.

**3. Examples**

Example 1 - Toothpaste compositions

Toothpaste samples 1 to 9 were produced according to the following procedure using the ingredients and amounts compiled in Table 1 below.

Step A: Sorbitol (70 % Sorbitol, Georges Walther AG, Switzerland) and cellulose gum (Sigma-Aldrich, Switzerland) were mixed in a beaker. Sodium monofluorophosphate (Phoskadent Na 211, BK Giulini, Germany) (if present), sodium fluoride (Phoskadent SF, BK Giulini, Germany) (if present), sodium benzoate (Georges Walther AG, Switzerland), and sodium saccharine (Omya Hamburg GmbH, Germany) were added to the sorbitol and cellulose gum in the beaker and were mixed under strong agitation. Then the water was added and mixed until a homogenous mixture was obtained.

Step B: SRCC 1 or SRCC 2 or Nano-HAP or Micro-HAP, respectively, and silica and titanium dioxide were added to the mixture of step A. The mixture was homogenized until a smooth mixture was obtained.

Step C: The cocamidopropyl betaine (Galaxy CAPD, Omya Hamburg GmbH, Germany) was added to the mixture of step B. The mixture was stirred slowly until smooth mixture was obtained.

Step D: The surfactant sodium lauryl sulphate (Galaxy 796G, Omya Hamburg GmbH, Germany) was added in form of a 25% solution to the mixture of step C under slow agitation, optionally under vacuum.

Step E: 0.8 wt.-% spearmint aroma (spearmint oil, Omya Hamburg GmbH, Germany) was added to the mixture of step D under slow agitation.

Table 1: Ingredients and amounts of toothpaste samples 1 to 9. The percentages refer to weight percentages based on the total weight of the final composition.

| Step | Ingredients | Amount [wt.-%] | | | | | | | | |
|---|---|---|---|---|---|---|---|---|---|---|
| | Sample number | 1 (comp.) | 2 (comp.) | 3 (comp.) | 4 (comp.) | 5 (comp.) | 6 (comp.) | 7 | 8 | 9 |
| A | Sorbitol | 30.00 | 30.00 | 30.00 | 30.00 | 30.00 | 30.00 | 30.00 | 30.00 | 30.00 |
| | Aqua | add.100 | add.100 | add.100 | add.100 | add.100 | add.100 | add.100 | add.100 | add.100 |
| | Sodium Monofluorophosphate | | | | | | | | 1.10 | |
| | Sodium Fluoride | | | 0.32 | | 0.32 | | 0.32 | | |
| | Cellulose Gum | 0.70 | 0.55 | 0.55 | 0.55 | 0.55 | 0.70 | 0.55 | 0.55 | 0.70 |
| | Sodium Benzoate | 0.20 | 0.20 | 0.20 | 0.20 | 0.20 | 0.20 | 0.20 | 0.20 | 0.20 |
| | Sodium Saccharin | 0.20 | 0.20 | 0.20 | 0.20 | 0.20 | 0.20 | 0.20 | 0.20 | 0.20 |
| B | SRCC 1 | | | | | | | 5.00 | 5.00 | 5.00 |
| | SRCC 2 | | 5.00 | 5.00 | | | | | | |
| | Nano-HAP | 5.00 | | | | | | | | |
| | Micro-HAP | | | | | | 5.00 | | | |
| | Silica | 20.00 | 20.00 | 20.00 | 20.00 | 20.00 | 20.00 | 20.00 | 20.00 | 20.00 |
| | Titanium Dioxide | 0.50 | 0.50 | 0.50 | 0.50 | 0.50 | 0.50 | 0.50 | 0.50 | 0.50 |
| C | Cocamidopropyl Betaine | 1.25 | 1.25 | 1.25 | 1.25 | 1.25 | 1.25 | 1.25 | 1.25 | 1.25 |
| D | Sodium Lauryl Sulfate | 1.15 | 1.15 | 1.15 | 1.15 | 1.15 | 1.15 | 1.15 | 1.15 | 1.15 |
| E | Spearmint aroma | 0.80 | 0.80 | 0.80 | 0.80 | 0.80 | 0.80 | 0.80 | 0.80 | 0.80 |
| | | **100.00** | **100.00** | **100.00** | **100.00** | **100.00** | **100.00** | **100.00** | **100.00** | **100.00** |

Example 2 - Remineralisation study

The study sought to compare the remineralisation potential of surface-reacted calcium carbonate-containing toothpastes pastes compared to commercially available toothpastes containing hydroxyapatite (HAP) in the presence and absence of fluoride, for which the benefits are well established. The design was based on an *in vitro* model published by Yu et al., "Effect of nano-hydroxyapatite concentration on the remineralization of initial enamel lesion in vitro", Biomedical Materials, 4 (2009), 034104, which also sought to demonstrate the remineralisation potential of suspensions of nano-hydroxyapatite. Results of this study showed how increasing HAP concentration brought about increased surface microhardness recovery (%SMHR) after 12 days of pH cycling and daily exposure to product treatments.

### 1. *Preparation of teeth samples*

### 1.1 *Colour pre-screen*

The colour of extracted human teeth was pre-screened using a CR-321 Chroma Meter (Konica Minolta, Japan) to identify teeth with $b^*$ values > 12. Teeth were selected from stocks stored in 0.1% thymol.

### 1.2 *Sample preparation*

Selected teeth were prepared according to the following method.

- The back of the tooth was abraded away until a slab approximately 3-4 mm thick was produced.
- The teeth were trimmed to enable them to be set into cuvettes, wherein the enamel surface was neither abraded nor polished.
- 4 groves were cut into the dentine layer to facilitate bonding of the sample with the acrylic.
- All dentine were painted with nail varnish to prevent exposure to test products.
- Cuvettes were cut to a height of 9 mm.
- Enamel blocks were set into the center of the cuvette (enamel surface exposed) using acrylic.
- One orientation mark was placed onto one side of the cuvette.
- The enamel blocks were stored in 0.01 M PBS solution.

### 1.3 *Baseline L\*a\*b\**

The baseline $L^*a^*b^*$ values of the enamel blocks were measured as described in section 2.5 below.

### 1.4 *Preparation of study solutions*

Remineralization and demineralization solution were prepared in advance.

Demineralization solution

A demineralization solution with the following concentration of chemicals was prepared:

- 50 mM acetic acid
- 2.2 mM calcium nitrate
- 2.2 mM potassium phosphate monobasic
- 0.1 ppm sodium fluoride

The final pH of the solution was adjusted to pH 4.5 with NaOH.

Remineralization solution

Remineralization solution with the following concentration of chemicals was prepared:

- 20 mM HEPES (4-(2-hydroxyethyl)-1-piperazineethanesulfonic acid)
- 1.5 mM calcium chloride
- 0.9 mM potassium phosphate monobasic
- 130 mM potassium chloride

- 1 mM sodium azide

The final pH of the solution was adjusted to pH 7.0 with KOH.

### 1.5 Initial demineralization

The enamel blocks were demineralized according to the steps below:

- The enamel blocks were individually demineralized in 8 ml of demineralization solution (see section 1.4).
- The enamel blocks were inserted into the oven set to 37°C for 1 hour.
- The enamel blocks were rinsed with deionized water for 2-3 minutes.

### 1.6 Post demineralization L*a*b*

The post demineralization L*a*b* values of the enamel blocks were measured as described in section 2.5 below.

### 2. Treatment of teeth samples

Some modification of the test parameters was necessary in order to take account of the fact that it was intended to test formulated toothpastes rather than suspensions of pure hydroxyapatite (HAP). For example, toothpaste slurries were made in the ratio 1 part toothpaste to 2 parts water and exposure times were increased from 3 minutes to 5 minutes. The duration of the test was also shortened to 3 days since it was anticipated that any product differences should be evident by this time-point. Surface microhardness was assessed after 3 days.

A short pilot was carried out to verify that the demineralisation protocol described by the published paper led to approximately 50% reduction in SMH compared to baseline. According to the paper, the required exposure time to acid is 72 hours at 37°C. However, we discovered that too much enamel softening occurred after 72 hours exposure to acid and that only 1 hour was actually necessary. This revision was therefore implemented for the full-scale study.

The enamel blocks were subjected to 3 days treatment and pH cycling as summarized in Table 2 below.

### 2.1 Treatment summary

Table 2: Treatment summary.

| Immersion | Duration | Specific Detail |
| --- | --- | --- |
| Specific slurry treatment applied | 5 min | 37°C agitated |
| Remin solution | 55 min | 37°C agitated |
| Specific slurry treatment applied | 5 min | 37°C agitated |
| Remin solution | 55 min | 37°C agitated |
| Demin solution | 1 hour | 37°C static |
| Remin solution | 2 hours | 37°C agitated |
| Specific slurry treatment applied | 5 min | 37°C agitated |
| Remin solution | 55 min | 37°C agitated |
| Specific slurry treatment applied | 5 min | 37°C agitated |
| Remin solution | overnight | 37°C agitated |

See section 2.4 below for detailed treatment description.

### 2.2 Block preparation

The enamel blocks were attached in a cluster. The cluster of blocks was placed inside a weighing boat for all immersions with exposed enamel surfaces facing upwards.

*2.3 Toothpaste slurry preparation*

- 220 ml slurries of the different toothpastes listed in Example 1 were prepared on the morning of each treatment day comprising 1 part toothpaste to 2 parts deionized water, e.g. 10.0g (+ 0.05g) of toothpaste to 20.0g (+ 0.05g) of DI water.
- The slurries were mixed thoroughly using an end-over mixer before being applied.
- 50 ml of each slurry was used for each treatment, which was decanted from the main stock slurry.

*2.4 Daily treatment and pH cycling*

All cycling immersions took place inside an incubator set to 37°C.

Treatment and remineralization immersions were agitated using a Stuart Plate Shaker set to 250 rpm, demineralization immersions were applied static.

The enamel blocks were stored in 0.01 M PBS solution when they are not being treated, e.g. on weekend storage.

The following method was followed for each of the 3 cycling days.

1. The blocks were immersed for 5 minutes in 50 mL of the appropriate slurry.
2. The blocks were rinsed with DI water to ensure all traces of slurry are removed.
3. The blocks were immersed in 50 ml of remineralization solution for 55 minutes.
4. The blocks were rinsed with DI water to ensure all traces of remineralization solution are removed.
5. The blocks were immersed for 5 minutes in 50 ml of the appropriate slurry.
6. The blocks were rinsed with DI water to ensure all traces of slurry are removed.
7. The blocks were immersed in 50 ml of remineralization solution for 55 minutes.
8. The blocks were rinsed with DI water to ensure all traces of remineralization solution are removed.
9. The blocks were immersed in 50 ml of demineralization solution for 60 minutes.
10. The blocks were rinsed with DI water to ensure all traces of demineralization solution are removed.
11. The blocks were immersed in 50 ml of remineralization solution for 120 minutes.
12. The blocks were rinsed with DI water to ensure all traces of remineralization solution are removed.
13. The blocks were immersed for 5 minutes in 50 ml of the appropriate slurry.
14. The blocks were rinsed with DI water to ensure all traces of slurry are removed.
15. The blocks were immersed in 50 ml of remineralization solution for 55 minutes.
16. The blocks were rinsed with DI water to ensure all traces of remineralization solution are removed.
17. The blocks were immersed for 5 minutes in 50 ml of the appropriate slurry.
18. The blocks were rinsed with DI water to ensure all traces of slurry are removed.
19. The blocks were immersed in 50 ml of remineralization solution overnight.
20. The blocks were rinsed with DI water to ensure all traces of remineralization solution are removed.

The blocks were subjected to four daily exposures of toothpaste slurry, each of 5 minutes duration, in between immersions in various remineralisation and demineralisation solutions. Toothpaste slurries containing NaMFP (sodium monofluorophosphate) also had alkaline phosphatase added in order to hydrolyse a proportion of the NaMFP and release free fluoride. Efficacy was assessed after 3 days pH cycling by re-measuring SMH and calculating mean percentage SMH recovery (%SMHR). Five indents were placed and measured per specimen, at each time point.

The average of the 5 SMH measurements was taken at each time point. The percentage surface microhardness recovery (SMHR) was calculated as:

•

$$\%SMHR = 100[(SMH_n - SMH_{post\ demin})/(SMH_{baseline} - SMH_{post\ demin})]$$

where n=3 days

Statistical analyses were conducted using SAS/STAT® software. Differences were assessed using an Analysis of Variance (ANOVA). The Tukey test for multiple comparisons was used. The results are shown in Fig. 1 and in Table 3 below.

Table 3: Results of enamel remineralisation tests.

| Toothpaste sample | Treatment group | SMHR after 3 days [%] |
|---|---|---|
| 1 (comp.) | TP with 5% nano-HAP + silica | 8 |
| 2 (comp.) | TP with 5% SRCC 2 + silica without fluoride | -8 |
| 3 (comp.) | TP with 5% SRCC 2 +silica + NaF | 50 |
| 4 (comp.) | TP with silica without fluoride | 18 |
| 5 (comp.) | TP with silica+ NaF | 59 |
| 6 (comp.) | TP with 5% micro-HAP + silica without fluoride | -5 |
| 7 | TP with 5% SRCC 1 + silica + NaF | 68 |
| 8 | TP with 5% SRCC 1 + silica + NaMFP | 58 |
| 9 | TP with 5% SRCC 1 + silica without fluoride | 81 |

The results shown in Fig. 1 confirm that the inventive compositions 7 to 9 show excellent remineralisation effects. Even in the absence of NaF (sodium fluoride) or NaMFP (sodium monofluorophosphate), a toothpaste containing 5% SRCC 1 and silica was able to bring about 80% surface re-hardening after 3 days of treatment. The size of the benefit was at least as good as other formulations containing fluoride (see comparative samples 3 and 5). Moreover, the remineralisation effect was even higher in the absence of any fluoride compound (see sample 9).

Toothpaste compositions containing nano- or micro-HAP were less effective (see comparative samples 1, 2, 4, and 6). It was also found that the comparative toothpaste samples 2 and 3 containing SRCC 2 do not show the same remineralisation effect as the inventive toothpastes. The benefits SRCC 2 shows when used in combination with fluoride (comparative sample 3) are presumably attributable to the fluoride alone.

Example 3 - Whitening study

This study was carried out to show that a surface-reacted calcium carbonate-containing toothpastes paste also shows an effect in whitening of teeth.

The set up was the same as described in Example 2 for the remineralisation of teeth under point 1. Preparation of teeth and point 2.Treatment of teeth samples.

The toothpaste treatment and pH cycling regime was carried out over a period of 12 days using toothpaste samples 1 (comparative), sample 7 (inventive) and a control of demineralized water. After this time as well as after the demineralization the L*a*b* values were measured.

*2.5 L*a*b* value measurement*

CIE *L*a*b** (CIELAB) is a color space specified by the International Commission on Illumination. It describes all the colors visible to the human eye and was created to serve as a device-independent model to be used as a reference.

The three coordinates of CIELAB represent the lightness of the color (***L***\* = 0 yields black and ***L*** \* = 100 indicates diffuse white; specular white may be higher), its position between red/magenta and green (***a***\*, negative values indicate green while positive values indicate magenta) and its position between yellow and blue (***b***\*, negative values indicate blue and positive values indicate yellow). The asterisk (\*) after *L, a* and *b* are pronounced *star* and are part of the full name, since they represent *L*, a* and b*,* to distinguish them from Hunter's *L, a,* and *b.*

The measurements take place at room temperature with enamel in a hydrated state (stored overnight in 0.1M PBS solution to rehydrate) and by using a Minolta CR321 chromameter.

The L*a*b* values were measured as follows:

1). The chromameter is placed into a jig.
2). Tooth samples are removed from PBS and dabbed dry.
3). An orientation mark of the block is placed away from the chromameter.
4). The L*a*b* values are measured at position 1 with the measuring head pressed against the tooth surface.
5). The block will be rotated 90 degrees and the L*a*b* values are read at position 2.
6) Step 5 is repeated until all orientations have been measured.

The measured values for b* after initial demineralisation (id) and 12 day treatment (12d) are listed for toothpaste samples 1 and 7 as well as the control consisting of demineralized water below in Table 4.

Table 4: Results of L*a*b* value measurement.

| Toothpaste sample | Treatment group | b*(id) | b*(12d) |
|---|---|---|---|
| 1 (comp.) | TP with 5% nano-HAP + silica | 14.3 | 11.8 |
| 7 (inventive) | TP with 5% SRCC 1 + silica + NaF | 14.1 | 11.2 |
| Control | Demineralized water | 14.2 | 10.0 |

The starting value of b* for all three treatment groups after initial demineralization was in the region of 14 indicating a strong bias to a yellow hue. Following the 12 days of treatment and pH cycling all three groups display a significantly less yellowing with b* values reducing by 2.5 (Sample 1) to 4.2 (water control) units. These results appear to support the idea that remineralized tooth enamel can appear whiter than the non-remineralized enamel.

The interesting finding here is that the deionized water control is significantly less yellow at the end of 12 days treatment than the two toothpaste groups. Without being bound to any theory the inventor believes that this may be due to the continued demineralisation and advancement of white spot lesion formation.

Similar trends were also obtained for the L* values. However, since the baseline trends for the three test groups were different from the beginning, the delta L* values are more meaningful. The delta L* values (L*(12d) - L*(id)) for the three treatment groups are listed in Table 5 below.

Table 5: Delta L* values.

| Toothpaste sample | Treatment group | Delta L* |
|---|---|---|
| 1 (comp.) | TP with 5% nano-HAP + silica | -1.8 |
| 7 (inventive) | TP with 5% SRCC 1 + silica + NaF | -2.8 |
| Control | Demineralized water | -3.7 |

**Claims**

1. An oral care composition comprising
   silica in an amount from 6 to 40 wt.-%, based on the total weight of the composition, and
   a surface reacted calcium carbonate in an amount from 1 to 40 wt.-%, based on the total weight of the composition, wherein the surface-reacted calcium carbonate is a reaction product of natural or synthetic calcium carbonate with carbon dioxide and at least one $H_3O^+$-ion donor, and
   the surface-reacted calcium carbonate is in form of particles having a volume determined median particle size $d_{50}$ from 2 to 5 $\mu$m, having a volume determined top cut particle size $d_{98}$ from 4 to 15 $\mu$m, and a specific surface area from 55 to 110 m$^2$/g, measured using nitrogen and the BET method according to ISO 9277.

2. The oral care composition of claim 1, wherein the at least one $H_3O^+$-ion donor is selected from the group consisting of hydrochloric acid, sulphuric acid, sulphurous acid, phosphoric acid, citric acid, oxalic acid, acetic acid, formic acid, and mixtures thereof, preferably the at least one $H_3O^+$-ion donor is selected from the group consisting of hydrochloric acid, sulphuric acid, sulphurous acid, phosphoric acid, oxalic acid, and mixtures thereof, and more preferably the at least one $H_3O^+$-ion donor is phosphoric acid.

3. The oral care composition of any one of the preceding claims, wherein the surface-reacted calcium carbonate has a volume determined median particle size $d_{50}$ from 2.4 to 4.5 $\mu$m, preferably from 2.5 to 4.0 $\mu$m, and most preferably from 2.8 to 3.5 $\mu$m.

4. The oral care composition of any one of the preceding claims, wherein the surface-reacted calcium carbonate has a volume determined top cut particle size $d_{98}$ from 5 to 13 $\mu$m, preferably from 7 to 12 $\mu$m, and most preferably from 9 to 11 $\mu$m.

5. The oral care composition of any one of the preceding claims, wherein the surface-reacted calcium carbonate has a specific surface area from 60 to 107 m$^2$/g, preferably from 70 to 105 m$^2$/g, and most preferably from 90 to 100

$m^2/g$, measured using nitrogen and the BET method according to ISO 9277.

6. The oral care composition of any one of the preceding claims, wherein the silica is present in an amount from 15 to 30 wt.-%, preferably from 15 to 25 wt.-%, and most preferably from 18 to 23 wt.-%, based on the total weight of the composition.

7. The oral care composition of any one of the preceding claims, wherein the surface-reacted calcium carbonate is present in an amount from 1 to 30 wt.-%, preferably from 2 to 15 wt.-%, more preferably from 3 to 10 wt.-%, and most preferably from 4 to 6 wt.-%, based on the total weight of the composition.

8. The oral care composition of any one of the preceding claims, wherein
the silica is present in an amount from 18 to 23 wt.-%, based on the total weight of the composition,
the surface reacted calcium carbonate is present in an amount from 4 to 6 wt.-%, based on the total weight of the composition,
the surface-reacted calcium carbonate is a reaction product of natural or synthetic calcium carbonate with carbon dioxide and phosphoric acid, and
the surface-reacted calcium carbonate is in form of particles having a volume determined median particle size $d_{50}$ from 2.8 to 3.5 $\mu$m, having a volume determined top cut particle size $d_{98}$ from 9 to 11 $\mu$m, and a specific surface area from 90 to 100 $m^2/g$, measured using nitrogen and the BET method according to ISO 9277.

9. The oral care composition of any one of the preceding claims, wherein the oral composition further comprises a fluoride compound, preferably the fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride.

10. The oral care composition of any one of the preceding claims, wherein the oral care composition further comprises an additional remineralisation and/or whitening agent, preferably selected from the group consisting of hydroxylapatite, nano-hydroxylapatite, calcium carbonate, amorphous calcium carbonate, and combinations thereof with casein phospholipids, hydrogen peroxide, carbamide peroxide, or fluoride compounds, and mixtures thereof.

11. The oral care composition of any one of the preceding claims, wherein the oral care composition is a toothpaste, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash, preferably a chewable gum, a lozenge, a toothpaste, a toothpowder, or a mouthwash, and most preferably a toothpaste.

12. The oral care composition of any one of the preceding claims, wherein at least one active agent is associated with the surface-reacted calcium carbonate, preferably the active agent is at least one additional desensitizing agent, and more preferably the at least one additional desensitizing agent is selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof.

13. The oral care composition of any one of the preceding claims, wherein the oral care composition has a pH between 7.5 and 10, preferably between 8 and 9.

14. The oral care composition of any one of the preceding claims, wherein the surface-reacted calcium carbonate is obtained by a process comprising the steps of:

a) providing an aqueous suspension of natural or synthetic calcium carbonate, wherein the aqueous suspension has a solids content in the range of 5 to 25 wt.-%, based on the weight of the aqueous suspension, and the amount of natural or synthetic calcium carbonate having a weight based particle size of less than 1 $\mu$m is at least 80 wt.-%, based on the total amount of natural or synthetic calcium carbonate, and
b) adding at least one $H_3O^+$-ion donor to the suspension of step a), and
c) treating the suspension of step a) with carbon dioxide before, during or after step b), wherein the carbon dioxide is formed in-situ by the $H_3O^+$-ion donor treatment and/or is supplied from an external source.

**15.** An oral care composition according to any one of the preceding claims for use in remineralisation and/or whitening of teeth.

**Patentansprüche**

**1.** Mundpflegezusammensetzung, umfassend
Siliciumdioxid in einer Menge von 6 bis 40 Gew.-%, bezogen auf das Gesamtgewicht der Zusammensetzung, und ein oberflächenreagiertes Calciumcarbonat in einer Menge von 1 bis 40 Gew.-%, bezogen auf das Gesamtgewicht der Zusammensetzung,
wobei das oberflächenreagierte Calciumcarbonat ein Reaktionsprodukt von natürlichem oder synthetischem Calciumcarbonat mit Kohlendioxid und mindestens einem $H_3O^+$-Ionendonor ist, und
das oberflächenreagierte Calciumcarbonat in Form von Partikeln, die eine volumenbestimmte mittlere Partikelgröße $d_{50}$ von 2 bis 5 $\mu$m aufweisen, vorliegt, die eine volumenbestimmte Oberschnitt-Partikelgröße $d_{98}$ von 4 bis 15 $\mu$m aufweisen, und eine spezifische Oberfläche von 55 bis 110 m$^2$/g, gemessen unter Verwendung von Stickstoff und dem BET-Verfahren gemäß ISO 9277.

**2.** Mundpflegezusammensetzung nach Anspruch 1, wobei der mindestens eine $H_3O^+$-Ionendonor ausgewählt ist aus der Gruppe, bestehend aus Salzsäure, Schwefelsäure, Schwefligsäure, Phosphorsäure, Zitronensäure, Oxalsäure, Essigsäure, Ameisensäure und Mischungen davon, wobei der mindestens eine $H_3O^+$-Ionendonor vorzugsweise ausgewählt ist aus der Gruppe, bestehend aus Salzsäure, Schwefelsäure, Schwefligsäure, Phosphorsäure, Oxalsäure und Mischungen davon und wobei der mindestens eine $H_3O^+$-Ionendonor mehr bevorzugt Phosphorsäure ist.

**3.** Mundpflegezusammensetzung nach einem der vorstehenden Ansprüche, wobei das oberflächenreagierte Calciumcarbonat eine volumenbestimmte mittlere Partikelgröße $d_{50}$ von 2,4 bis 4,5 $\mu$m, vorzugsweise von 2,5 bis 4,0 $\mu$m und am meisten bevorzugt von 2,8 bis 3,5 $\mu$m aufweist.

**4.** Mundpflegezusammensetzung nach einem der vorstehenden Ansprüche, wobei das oberflächenreagierte Calciumcarbonat eine volumenbestimmte Oberschnitt-Partikelgröße $d_{98}$ von 5 bis 13 $\mu$m, vorzugsweise von 7 bis 12 $\mu$m und am meisten bevorzugt von 9 bis 11 $\mu$m aufweist.

**5.** Mundpflegezusammensetzung nach einem der vorstehenden Ansprüche, wobei das oberflächenreagierte Calciumcarbonat eine spezifische Oberfläche von 60 bis 107 m$^2$/g, vorzugsweise von 70 bis 105 m$^2$/g und am meisten bevorzugt von 90 bis 100 m$^2$/g aufweist, gemessen unter Verwendung von Stickstoff und dem BET-Verfahren gemäß ISO 9277.

**6.** Mundpflegezusammensetzung nach einem der vorstehenden Ansprüche, wobei das Siliciumdioxid, bezogen auf das Gesamtgewicht der Zusammensetzung, in einer Menge von 15 bis 30 Gew.-%, vorzugsweise von 15 bis 25 Gew.-% und am meisten bevorzugt von 18 bis 23 Gew.-% vorliegt.

**7.** Mundpflegezusammensetzung nach einem der vorstehenden Ansprüche, wobei das oberflächenreagierte Calciumcarbonat, bezogen auf das Gesamtgewicht der Zusammensetzung, in einer Menge von 1 bis 30 Gew.-%, vorzugsweise von 2 bis 15 Gew.-%, mehr bevorzugt von 3 bis 10 Gew.-% und am meisten bevorzugt von 4 bis 6 Gew.-% vorliegt.

**8.** Mundpflegezusammensetzung nach einem der vorstehenden Ansprüche, wobei
das Siliciumdioxid, bezogen auf das Gesamtgewicht der Zusammensetzung, in einer Menge von 18 bis 23 Gew-% vorliegt,
das oberflächenreagierte Calciumcarbonat, bezogen auf das Gesamtgewicht der Zusammensetzung, in einer Menge von 4 bis 6 Gew.-% vorliegt,
wobei das oberflächenreagierte Calciumcarbonat ein Reaktionsprodukt von natürlichem oder synthetischem Calciumcarbonat mit Kohlendioxid und Phosphorsäure ist, und
das oberflächenreagierte Calciumcarbonat in Form von Partikeln mit einer volumenbestimmten mittleren Partikelgröße $d_{50}$ von 2,8 bis 3,5 $\mu$m vorliegt, mit einer volumenbestimmten Oberschnitt-Partikelgröße $d_{98}$ von 9 bis 11 $\mu$m, und einer spezifischen Oberfläche von 90 bis 100 m$^2$/g, gemessen unter Verwendung von Stickstoff und dem BET-Verfahren gemäß ISO 9277.

**9.** Mundpflegezusammensetzung nach einem der vorstehenden Ansprüche, wobei die Mundzusammensetzung weiter eine Fluoridverbindung umfasst, wobei die Fluoridverbindung ausgewählt ist aus der Gruppe, bestehend aus Natriumfluorid, Zinn(II)fluorid, Natriummonofluorphosphat, Kaliumfluorid, Kaliumzinnfluorid, Natriumfluorstannat, Zinnchlorfluorid, Aminfluorid und Mischungen davon, und wobei die Fluoridverbindung mehr bevorzugt Natriummonofluorphosphat und/oder Natriumfluorid ist.

**10.** Mundpflegezusammensetzung nach einem der vorstehenden Ansprüche, wobei die Mundpflegezusammensetzung weiter ein zusätzliches Remineralisations- und/oder Aufhellungsmittel umfasst, vorzugsweise ausgewählt aus der Gruppe, bestehend aus Hydroxylapatit, Nanohydroxylapatit, Calciumcarbonat, amorphem Calciumcarbonat und Kombinationen davon mit Casein-Phospholipiden, Wasserstoffperoxid, Carbamidperoxid oder Fluoridverbindungen und Mischungen davon.

**11.** Mundpflegezusammensetzung nach einem der vorstehenden Ansprüche, wobei die Mundpflegezusammensetzung eine Zahnpasta, ein Zahnpulver, ein Lack, ein Haftmittelgel, ein Zement, ein Harz, ein Spray, ein Schaum, ein Balsam, eine auf einem Mundstreifen oder einem bukkalen Haftmittelpflaster ausgeführte Zusammensetzung, eine kaubare Tablette, eine kaubare Pastille, ein kaubarer Gummi, eine Schmelztablette, ein Getränk oder eine Mundspülung, vorzugsweise ein kaubarer Gummi, eine Schmelztablette, eine Zahnpasta, ein Zahnpulver oder einer Mundspülung und am meisten bevorzugt eine Zahnpasta ist.

**12.** Mundpflegezusammensetzung nach einem der vorstehenden Ansprüche, wobei mindestens ein Wirkstoff mit dem oberflächenreagierten Calciumcarbonat assoziiert ist, wobei der Wirkstoff vorzugsweise mindestens ein zusätzliches Desensibilisierungsmittel ist und mehr bevorzugt wobei das mindestens eine zusätzliche Desensibilisierungsmittel ausgewählt ist aus der Gruppe, bestehend aus Kaliumnitrat, Gluteraldehyde, Silbernitrat, Zinkchlorid, Strontiumchloridhexahydrat, Natriumfluorid, Zinn(II)fluorid, Strontiumchlorid, Strontiumacetat, Arginin, Hydroxylapatit, Calciumnatriumphosphosilikat, Kaliumoxalat, Calciumphosphat, Calciumcarbonat, bioaktiven Gläsern und Mischungen davon.

**13.** Mundpflegezusammensetzung nach einem der vorstehenden Ansprüche, wobei die Mundpflegezusammensetzung einen pH-Wert zwischen 7,5 und 10, vorzugsweise zwischen 8 und 9 aufweist.

**14.** Mundpflegezusammensetzung nach einem der vorstehenden Ansprüche, wobei das oberflächenreagierte Calciumcarbonat erhalten wird durch einen Prozess, umfassend die Schritte des:

a) Bereitstellens einer wässrigen Suspension von natürlichem oder synthetischem Calciumcarbonat, wobei die wässrige Suspension einen Feststoffgehalt im Bereich von 5 bis 25 Gew.-%, bezogen auf das Gewicht der wässrigen Suspension, aufweist, und die Menge an natürlichem oder synthetischen Calciumcarbonat mit einer gewichtsbasierten Partikelgröße von weniger als 1 $\mu$m mindestens 80 Gew.-% beträgt, basierend auf der Gesamtmenge von natürlichem oder synthetischem Calciumcarbonat, und
b) Zugebens mindestens eines $H_3O^+$-Ionendonors zu der Suspension von Schritt a), und
c) Behandelns der Suspension von Schritt a) mit Kohlendioxid vor, während oder nach Schritt b), wobei das Kohlendioxid durch die $H_3O^+$-Ionendonor-Behandlung in-situ gebildet wird und/oder aus einer externen Quelle zugeführt wird.

**15.** Mundpflegezusammensetzung nach einem der vorstehenden Ansprüche zur Verwendung bei der Remineralisation und/oder dem Aufhellen von Zähnen.

**Revendications**

**1.** Composition de soins buccaux comprenant
de la silice en quantité de 6 à 40 % en poids sur la base du poids total de la composition ; et
un carbonate de calcium qui réagit en surface en quantité de 1 à 40 % en poids sur la base du poids total de la composition,
dans laquelle le carbonate de calcium qui réagit en surface est un produit réactionnel de carbonate de calcium naturel ou synthétique avec du dioxyde de carbone et au moins un donneur d'ions $H_3O^+$ et
le carbonate de calcium qui réagit en surface se présente sous la forme de particules ayant une taille particulaire moyenne déterminée en volume $d_{50}$ de 2 à 5 $\mu$m, ayant une taille particulaire de coupe supérieure déterminée en volume $d_{98}$ de 4 à 15 $\mu$m et une surface spécifique de 55 à 110 m$^2$/g mesurée en utilisant de l'azote et le procédé BET selon la norme ISO 9277.

**2.** Composition de soins buccaux selon la revendication 1, dans laquelle le au moins un donneur d'ions $H_3O^+$ est choisi dans le groupe constitué de l'acide chlorhydrique, de l'acide sulfurique, de l'acide sulfureux, de l'acide phosphorique, de l'acide citrique, de l'acide oxalique, de l'acide acétique, de l'acide formique et de leurs mélanges, de préférence le au moins un donneur d'ions $H_3O^+$ est choisi dans le groupe constitué de l'acide chlorhydrique, de l'acide sulfurique, de l'acide sulfureux, de l'acide phosphorique, de l'acide oxalique et de leurs mélanges et, mieux encore, le au moins un donneur d'ions $H_3O^+$ est l'acide phosphorique.

**3.** Composition de soins buccaux selon l'une quelconque des revendications précédentes, dans laquelle le carbonate de calcium qui réagit en surface a une taille particulaire moyenne déterminée en volume $d_{50}$ de 2,4 à 4,5 $\mu$m, de préférence de 2,5 à 4,0 $\mu$m et, mieux encore, de 2,8 à 3,5 $\mu$m.

**4.** Composition de soins buccaux selon l'une quelconque des revendications précédentes, dans laquelle le carbonate de calcium qui réagit en surface à une taille particulaire en coupe supérieure déterminée en volume $d_{98}$ de 5 à 13 $\mu$m, de préférence de 7 à 12 $\mu$m, mieux encore, de 9 à 11 $\mu$m.

**5.** Composition de soins buccaux selon l'une quelconque des revendications précédentes, dans laquelle le carbonate de calcium qui réagit en surface a une surface spécifique de 60 à 107 m$^2$/g, de préférence de 70 à 105 m$^2$ /g et, mieux encore, de 90 à 100 m$^2$/g, mesurée en utilisant de l'azote et le procédé BET selon la norme ISO 9277.

**6.** Composition de soins buccaux selon l'une quelconque des revendications précédentes, dans laquelle la silice est présente en quantité de 15 à 30 % en poids, de préférence de 15 à 25 % en poids, mieux encore de 18 à 23 % en poids sur la base du poids total de la composition.

**7.** Composition de soins buccaux selon l'une quelconque des revendications précédentes, dans laquelle le carbonate de calcium qui réagit en surface est présent en quantité de 1 à 30 % en poids, de préférence de 2 à 15 % en poids, mieux encore de 3 à 10 % en poids et bien mieux encore de 4 à 6 % en poids sur la base du poids total de la composition.

**8.** Composition de soins buccaux selon l'une quelconque des revendications précédentes, dans laquelle :

la silice est présente en quantité de 18 à 23 % en poids sur la base du poids total de la composition ; et
le carbonate de calcium qui réagit en surface est présent en quantité de 4 à 6 % en poids sur la base du poids total de la composition,
le carbonate de calcium qui réagit en surface est un produit réactionnel de carbonate de calcium naturel ou synthétique avec du dioxyde de carbone et de l'acide phosphorique et
le carbonate de calcium qui réagit en surface se présente sous la forme de particules ayant une taille particulaire moyenne déterminée en volume $d_{50}$ de 2,8 à 3,5 $\mu$m, ayant une taille particulaire de coupe supérieure déterminée en volume $d_{98}$ de 9 à 11 $\mu$m et une surface spécifique de 90 à 100 m$^2$/g mesurée en utilisant de l'azote et le procédé BET selon la norme ISO 9277.

**9.** Composition de soins buccaux selon l'une quelconque des revendications précédentes, dans laquelle la composition buccale comprend en outre un composé de fluorure, de préférence le composé de fluorure est choisi dans le groupe constitué du fluorure de sodium, du fluorure stanneux, du monofluorophosphate de sodium, du fluorure de potassium, du fluorure stanneux de potassium, du fluorostannate de sodium, du chlorofluorure stanneux, de fluorure d'amine et de leurs mélanges et, mieux encore, le composé de fluorure est le monofluorophosphate de sodium et/ou le fluorure de sodium.

**10.** Composition de soins buccaux selon l'une quelconque des revendications précédentes, dans laquelle la composition de soins buccaux comprend en outre un agent supplémentaire de reminéralisation et/ou de blanchiment, de préférence choisi dans le groupe constitué de l'hydroxyapatite, de la nano-hydroxyapatite, du carbonate de calcium, du carbonate de calcium amorphe et de leurs combinaisons avec des phospholipides de caséine, du peroxyde d'hydrogène, de peroxyde de carbamide ou des composés de fluorure et leurs mélanges.

**11.** Composition de soins buccaux selon l'une quelconque des revendications précédentes, dans laquelle la composition de soins buccaux est une pâte dentifrice, une poudre dentaire, un vernis, un gel adhésif, un ciment, une résine, un jet pulvérisé, une mousse, un baume, une composition appliquée sur une bande buccale ou une pièce adhésive buccale, un comprimé à mâcher, une pâte à mâcher, une gomme à mâcher, une pastille, une boisson, un bain de bouche, de préférence un chewing-gum, une pastille, une pâte dentifrice, une poudre dentaire ou un bain de bouche et, mieux encore, une pâte dentifrice.

**12.** Composition de soins buccaux selon l'une quelconque des revendications précédentes, dans laquelle au moins un agent actif est associé au carbonate de calcium qui réagit en surface, de préférence l'agent actif est au moins un agent de désensibilisation supplémentaire et, mieux encore, le au moins un agent désensibilisant supplémentaire est choisi dans le groupe constitué du nitrate de potassium, du gluteraldéhyde, du nitrate d'argent, du chlorure de zinc, du chlorure de strontium hexahydraté, du fluorure de sodium, du fluorure stanneux, du chlorure de strontium, de l'acétate de strontium, de l'arginine, de l'hydroxyapatite, du phosphosilicate de calcium et de sodium, de l'oxalate de potassium, du phosphate de calcium, du carbonate de calcium, des verres bioactifs et de leurs mélanges.

**13.** Composition de soins buccaux selon l'une quelconque des revendications précédentes, dans laquelle la composition de soins buccaux à un pH entre 7,5 et 10, de préférence entre 8 et 9.

**14.** Composition de soins buccaux selon l'une quelconque des revendications précédentes, dans laquelle le carbonate de calcium qui réagit en surface est obtenu par un procédé comprenant les étapes consistant à :

a) fournir une suspension aqueuse de carbonate de calcium naturel ou synthétique, dans laquelle la suspension aqueuse a une teneur en solides dans la plage de 5 à 25 % en poids sur la base du poids de la suspension aqueuse et la quantité de carbonate de calcium naturel ou synthétique ayant une taille particulaire à base pondérale de moins de 1 $\mu$m est d'au moins 80 % en poids sur la base de la quantité totale de carbonate de calcium naturel ou synthétique et
b) ajouter au moins un donneur d'ions $H_3O^+$ à la suspension de l'étape a) et
c) traiter la suspension de l'étape a) avec du dioxyde de carbone avant, pendant ou après l'étape b), dans laquelle le dioxyde de carbone est formé in situ par le traitement au donneur d'ions $H_3O^+$ et/ou est fourni par une source externe.

**15.** Composition de soins buccaux selon l'une quelconque des revendications précédentes pour utilisation dans la reminéralisation et/ou le blanchiment des dents.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070183984 A1
- WO 2012143220 A1
- WO 2013034421 A2
- WO 2012031786 A2
- WO 2015150011 A1
- WO 2015140308 A1
- WO 0039222 A1
- US 20040020410 A1
- EP 2264108 A1
- EP 1974806 A1
- EP 1982759 A1
- EP 1974807 A1
- WO 2010037753 A1
- EP 2070991 A1

**Non-patent literature cited in the description**

- **HORNBY et al.** *International Dental Journal,* 2009, vol. 59, 325-331
- **BATH-BALOGH ; FEHRENBACH.** Illustrated Dental Embryology, Histology, and Anatomy. Elsevier, 2011, 180
- Tooth bleaching. Wikipedia, The Free Encyclopedia. 05 February 2014
- **HARRIS, D. C.** Quantitative Chemical Analysis. W.H. Freeman & Co, 1991
- **GANE, P.A.C. ; KETTLE, J.P. ; MATTHEWS, G.P. ; RIDGWAY, C.J.** Void Space Structure of Compressible Polymer Spheres and Consolidated Calcium Carbonate Paper-Coating Formulations. *Industrial and Engineering Chemistry Research,* 1996, vol. 35 (5), 1753-1764
- **STIRNIMANN et al.** *International Journal of Pharmaceutics,* 2014, vol. 466, 266-275
- **YU et al.** Effect of nano-hydroxyapatite concentration on the remineralization of initial enamel lesion in vitro. *Biomedical Materials,* 2009, vol. 4, 034104